# EUROPÄISCHE PATENTSCHRIFT

(11) **EP 1 356 848 B1**
(45) Veröffentlichungstag und Bekanntmachung des Hinweises auf die Patenterteilung: **01.09.2004**
(21) Anmeldenummer: 02009073.4
(22) Anmeldetag: 23.04.2002
(51) Int. Cl.: A61N 5/06

(54) **Vorrichtung zur Behandlung der Haut mittels UV-Licht**
Device for treating the skin with ultraviolet light
Dispositif pour le traitement de la peau par lumière ultraviolette

(43) Veröffentlichungstag der Anmeldung: 29.10.2003
(73) Patentinhaber: Tui Laser AG, 82110 Germering (DE); Ockenfels, Hans Michael, Dr. Med., 63755 Alzenau (DE)
(72) Erfinder: Münker, Michael, 81375 München (DE); Ockenfels, Hans Michael, Dr., 63756 Alzenau/Unterfranken (DE)
(74) Vertreter: Schnekenbühl, Robert Matthias L.

(56) Entgegenhaltungen:
- EP-A- 0 765 674
- EP-A- 1 195 176
- WO-A-00/64537
- WO-A-02/13905
- WO-A-85/02532
- WO-A-99/17668
- US-A- 4 558 700
- US-A1- 2002 013 609

## Beschreibung

Die vorliegende Erfindung betrifft eine Vorrichtung zur Behandlung einer Oberfläche, insbesondere der tierischen oder menschlichen Haut, umfassend eine UVA-Strahlungsquelle, deren Strahlung in eine Lichtleiter eingekoppelt wird und eine UVB-Strahlungsquelle, deren Strahlung in denselben Lichtleiter oder einen weiteren Lichtleiter eingekoppelt wird, wobei die Strahlung der beiden Strahlungsquellen an einer Austrittsfläche aus dem einen Lichtleiter oder aus den Austrittsflächen der beiden Lichtleiter ausgekoppelt wird.

Es sind Vorrichtungen und Verfahren bekannt, bei denen eine Oberfläche, insbesondere die menschliche oder tierische Haut, mit Licht einer Wellenlänge bestrahlt wird. Derartige Verfahren werden bei einer Vielzahl von Indikationen, bei denen eine Bestrahlung angezeigt sein kann, angewandt, so z.B. zur Behandlung der Psoriasis, Vitiligo, Hypopigmentierung (Leukoderma), Granuloma anulare, Lichen planus, Alopecie areata, Neurodermitis, Acne etc. Das Licht kann eine UV-Lichtquelle wie eine UVA- oder UVB-Lichtquelle bzw. eine Lichtquelle, die UVA- und UVB-Licht kombiniert abgeben kann, enthalten. Üblicherweise wird dieses Licht von einer Lampe abgegeben, die die Haut großflächig bestrahlt.

Des weiteren sind Vorrichtungen und Verfahren bekannt, bei denen ein gepulster Laser UVB-Licht abgibt. Zur Erhöhung der Empfindlichkeit der Haut gegenüber einer Bestrahlung ist das verabreichen von Medikamenten, so genannter Photosensibilisatoren, wie z.B. Psoralens bekannt.

Aus der WO 99/17668 ist eine Vorrichtung zur Behandlung einer Oberfläche, insbesondere der tierischen oder menschlichen Haut, bekannt. Dabei wird eine UV-Strahlung als Behandlungsstrahlung zusammen mit einer Beleuchtungsstrahlung zum Ausleuchten der zu behandelnden Oberfläche mit sichtbarem Licht gemeinsam in einen Lichtleiter eingekoppelt und an der Austrittsfläche eines Behandlungsgriffstückes gemeinsam aus dem Lichtleiter ausgekoppelt.

Aus der WO 02/13905 ist eine gattungsbildende Vorrichtung bekannt, bei der sowohl UVA- als auch UVB-Strahlung von einer gemeinsamen Strahlungsquelle erzeugt und über Filter- bzw. Spiegelanordnungen in jeweils unterschiedliche Lichtleiter eingekoppelt werden.

Nachteilig an Vorrichtungen und Verfahren nach Stand der Technik ist ein meist vergleichsweise geringer Behandlungserfolg und teilweise erhebliche Nebenwirkungen insbesondere auf die gesunde Haut. Nebenwirkungen können von Färbungen und Sonnenbrand bis zu bleibender Schädigung, Vernarbung oder Hautkrebs reichen.

Der vorliegenden Erfindung liegt daher die Aufgabe zugrunde, eine Vorrichtung und ein Verfahren anzugeben, mit denen ein besserer Behandlungserfolg bei geringen Nebenwirkungen erzielt werden kann.

Dieses Problem wird durch eine Vorrichtung zur Behandlung einer Oberfläche, insbesondere der tierischen oder menschlichen Haut, umfassend eine UVA-Strahlungsquelle, deren Strahlung in eine Lichtleiter eingekoppelt wird und eine UVB-Strahlungsquelle, deren Strahlung in denselben Lichtleiter oder einen weiteren Lichtleiter eingekoppelt wird, wobei die Strahlung der beiden Strahlungsquellen an einer Austrittsfläche aus dem einen Lichtleiter oder aus den Austrittsflächen der beiden Lichtleiter ausgekoppelt wird, bei der die UVA-Strahlungsquelle und die UVB-Strahlungsquelle unterschiedliche Strahlungsquellen sind, deren Intensität unabhängig voneinander gewählt werden kann, gelöst.

Die Austrittsfläche für die Strahlung der ersten Strahlungsquelle und die weitere Austrittsfläche für die Strahlung der weiteren Strahlungsquelle kann dabei identisch sein, ebenso können die Austrittsflächen aber auch unmittelbar nebeneinander liegen. Als Strahlungsquelle kommen Lampen oder Laser oder dergleichen in Betracht. Die Austrittsfläche ist insbesondere als Handstück ausgeführt, welche die behandelnde Person manuell über die zu behandelnde Fläche führen kann.

Besonders vorteilhaft ist es, wenn nur ein Lichtleiter verwendet wird. Damit ist gemeint, dass sowohl die Strahlung der ersten Strahlungsquelle als auch die der zweiten Strahlungsquelle über den gleichen Lichtleiter geführt werden. Neben einer einfachen Herstellbarkeit der Vorrichtung resultiert daraus ein einfacherer, leichterer und damit einfacher zu handhabender Lichtleiter.

UVB-Licht ist Licht in einem Wellenlängenbereich von etwa 280nm bis 320nm. Als UVB-Lampe kommen hier z.B. Quecksilberdampflampen, Xenon-Hochdrucklampen sowie 308nm Excimer-Laser in Betracht. Vorzugsweise kann die UVB Strahlungsquelle Licht einer Wellenlänge von etwa 290 bis 320 nm abgeben. Licht dieser Wellenlänge wirkt bei Behandlung der menschlichen Haut insbesondere an deren Oberfläche und dringt nur bis in eine geringe Tiefe der Haut ein.

UVA-Licht ist Licht in einem Wellenlängenbereich von etwa 320nm bis etwa 400nm. Als UVA-Lampe kommen hier beispielsweise ähnliche Lampen wie im UVB Bereich sowie 351nm XeF Excimer-Laser, Argonionen-Laser, frequenzverdreifachte Nd-YAG-Laser mit 351nm sowie im Bereich von 360nm bis 400nm einstellbare frequenzverdoppelte Alexandritlaser in Betracht. UVA-Licht dringt auch in tiefere Hautschichten ein und ermöglicht so auch die Behandlung tieferer Hautschichten. Da die Strahlung der ersten Strahlungsquelle und die der zweiten Strahlungsquelle jeweils von unterschiedlichen Strahlungsquellen erzeugt werden, kann deren Intensität unabhängig voneinander gewählt werden. Dies ermöglicht es, das Verhältnis des insbesondere auf der Oberfläche der menschlichen Haut wirkenden UVB-Lichts und des auch in tieferen Hautregionen wirkenden UVA-Lichts beliebig abzustimmen. Je nach Art der zu behandelnden Erkrankung ist auf diese Weise eine beliebige Einstellung der Tiefe, in der die von der Vorrichtung abgegebene Strahlung wirkt, möglich.

Besonders vorteilhaft ist es, wenn die UVB Strahlungsquelle und/oder die UVA Strahlungsquelle ein Laser ist. Auf diese Weise ist es möglich, durch Auswahl geeigneter Laser eine beliebige Abstimmung der verwendeten Wellenlänge herbeizuführen. Insbesondere hat ein Laser gegenüber Lampen als Lichtquelle den Vorteil eine definierte Wellenlänge sowie eine genau einstellbare Beleuchtungsleistung zu ermöglichen. Als Laser kommen dabei beliebige gepulste oder nicht gepulste Laser in Betracht.

Besonders vorteilhaft ist es, wenn der UVB und/oder UVA Laser gepulst ist. Gepulste Laser ermöglichen hohe Leistungsdichten und lassen sich durch Verändern der Pulsfolge in ihrer Leistung besonders leicht regeln.

Bevorzugt ist die UVB Strahlungsquelle ein Laser mit einer Pulslänge von etwa 10ns bis 300ns. Diese Pulslänge ist mit einem XeCl-Laser realisierbar und hat sich im versuch als besonders effektiv bei der Behandlung der Psoriasis erwiesen.

Vorzugsweise ist die UVA Strahlungsquelle ein Laser mit einer Pulslänge von etwa 10ns bis 100 Millisekunden.

In einer Weiterbildung der Vorrichtung ist vorgesehen, dass die Pulse der UVB Strahlungsquelle und der UVA Strahlungsquelle als Impulsfolgen abgegeben werden können wobei eine Impulsfolge aus einem oder mehreren Impulsen bestehen kann und die Anzahl der Pulse für jede Strahlungsquelle unterschiedlich sein kann. Wenn nur eine Strahlungsquelle gepulst und die andere kontinuierlich betrieben wird kann die kontinuierlich betriebene Strahlungsquelle während der Dauer eines Pulses der anderen Strahlungsquelle auch unterbrochen werden, z.B. mechanisch über Chopper, Shutter oder Scanner oder elektro-optisch durch eine Kerr- oder Pockels-Zelle. Die Pulse der UVB sowie der UVA Strahlungsquelle können jeweils als Einzelimpuls oder als Folge mehrerer Impulse abgegeben werden. Mit der abwechselnden Abgabe der Impulsfolge der UVB Strahlungsquelle und der UVA Strahlungsquelle ist hier gemeint, dass nachdem beispielsweise die UVB Strahlungsquelle eine Folge von Impulsen abgegeben hat, nunmehr die UVA Strahlungsquelle eine Folge von Impulsen abgibt. Dabei können beliebig lange Pausen zwischen den beiden Impulsfolgen vorliegen. Nachdem beide Strahlungsquellen eine Impulsfolge abgegeben haben erfolgt nach einer beliebig kurzen oder langen Pause wieder die Abgabe der Impulsfolge der UVB Strahlungsquelle und darauf die Abgabe der Pulsfolge der UVA Strahlungsquelle und so weiter. Durch geeignete Auswahl der Impulslänge und der Anzahl der jeweils abgegebenen Impulse kann die Wirkung auf die Hautoberfläche und die Wirkung auf die tieferen Hautschichten beliebig gegeneinander variiert werden. Die Einstellung von Impulslängen und Impulsfolgen ist bei gängigen gepulsten Lasern besonders einfach regelbar.

In einer Weiterbildung der erfindungsgemäßen Vorrichtung ist vorgesehen, dass die UVB Strahlungsquelle ein Laser mit einer Wellenlänge von etwa 308 nm ist. Es kann sich hier um einen handelsüblichen XeCl Laser handeln.

In einer Weiterbildung der erfindungsgemäßen Vorrichtung ist vorgesehen, dass die UVA Strahlungsquelle ein UVA-Laser mit einer Wellenlänge größer als etwa 320 nm ist. Vorzugsweise kann die UVA Strahlungsquelle ein XeF- oder Ar-Ionen-Laser mit einer Wellenlänge von etwa 351 nm, ein NdYAG-Laser mit einer Wellenlänge von etwa 355 nm, ein durchstimmbarer Laser mit einer Wellenlänge von etwa 360 bis 430 nm.

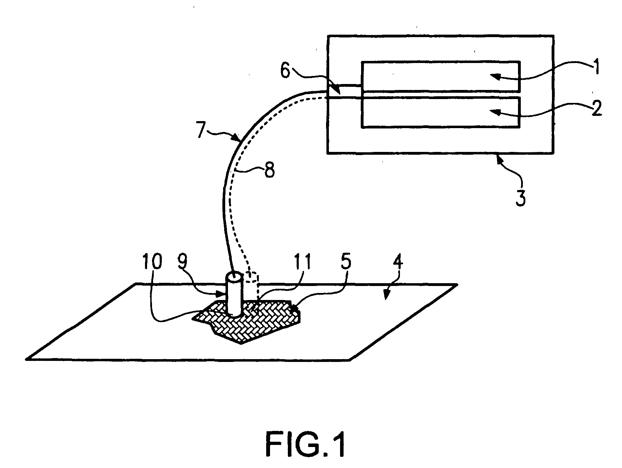

Im Folgenden wird ein Ausführungsbeispiel der Erfindung anhand der beiliegenden Zeichnungen erläutert. Dabei zeigt
- Fig. 1: eine Prinzipskizze einer erfindungsgemäßen Vorrichtung.

Eine UVB Strahlungsquelle 1, eine UVB-Lampe einer Wellenlänge von 290 nm bis 320 nm oder beispielsweise ein XeCL-Laser einer Wellenlänge von 308 nm, ist zusammen mit einer UVA Strahlungsquelle 2, einer UVA-Lampe oder einem UVA-Laser, in einem gemeinsamen Gehäuse 3 angeordnet. Die UVB Strahlungsquelle 1 und die UVA Strahlungsquelle 2 können alternativ auch in unterschiedlichen Gehäusen angeordnet sein. Die UVA Strahlungsquelle 2 kann beispielsweise ein XeF oder Ar-Ionen-Laser mit einer Wellenlänge von z. B. 351 nm und einer Pulslänge größer als 10ns sein. Alternativ kommt auch ein NdYAG-Laser, dreifach, mit einer Wellenlänge von 355 nm oder ein durchstimmbarer Laser Alexandrit, zweifach, mit einer Wellenlänge von 360 nm bis 430 nm und Pulslängen jeweils im Bereich von 10 Millisekunden bis 100 Millisekunden in Betracht. Die angegebenen Wellenlängen sind nur Anhalte, hier können auch für wesentlich längerwellige oder kürzerwellige Strahlungen in Frage kommen.

Die Strahlung der UVB Strahlungsquelle 1 wird an einer Einkoppelung 6 in einen Lichtleiter 7 eingekoppelt. Wird die gleiche Faser als Lichtleiter 7 benutzt, so müssen beide Strahlen zur Einkoppelung möglichst den gleichen Weg zur Oberfläche durchlaufen, die Strahlmitte ist im Idealfall senkrecht zur Oberfläche am Faserende. Die Fasereinkoppelung erfolgt dabei idealerweise durch Bündelung des Strahls mit einer Sammellinse in einem Winkel, der der numerischen Apertur der Faser entspricht. Die Strahlen werden dazu mit einer Linse oder mehreren separaten Linsen auf einen Punkt vor der Faser fokussiert, die Strahlen treffen dann beim auseinandergehen nach dem Fokus auf die Faser. Die Strahlungen aus beiden Lichtquellen werden auf einen Scanner mit beweglichem Spiegel geschickt, der beide Wellenlängen reflektiert und im Takt der Impulsfolgen mal den einen und mal den anderen Strahl in Richtung der Faser lenkt, wo diese eingekoppelt werden. Die fokussierende Linse und begrenzende Blende, die für die Einkopplung benötigt wird, kann entweder vor dem Scanner in jedem Strahl separat oder nach dem Scanner für beide Strahlen gemeinsam geschehen. Alternativ kann ein Spiegel für eine Wellenlänge reflektierend, für die andere Wellenlänge durchlässig sein. Der Strahlengang der einen Strahlquelle geht durch den Spiegel hindurch zum Lichtleiter 7 während der andere erst durch den Spiegel in die gemeinsame Richtung abgelenkt und in den Lichtleiter 7 eingekoppelt wird. Der Lichtleiter 7 kann ebenso ein Spiegelarm sein. Vorzugsweise werden gepulste Quellen eingesetzt, um die Belastung der Strahlführung zu verringern. Die Strahlung der UVA Strahlungsquelle 2 wird entsprechend entweder in den Lichtleiter 7 oder in einen UVA Lichtleiter 8 eingekoppelt. Vorzugsweise sind der Lichtleiter 7 und der weitere Lichtleiter 8 identisch, die Strahlung der UVB Strahlungsquelle 1 und die der UVA Strahlungsquelle 2 werden also über den gleichen Lichtleiter 7 geleitet.

Über ein Handstück 9 des Lichtleiters 7 wird die eingekoppelte Strahlung an einer Austrittsfläche 10 und entsprechend bei Verwendung eines weiteren Lichtleiters 8 an einer weiteren Austrittsfläche 11 ausgekoppelt. Dies bedeutet, dass das von der UVB Strahlungsquelle 1 und einer UVA Strahlungsquelle 2 abgegebene Strahlung an der Austrittsfläche 10 bzw. 11 austritt.

Insbesondere wenn die UVB Strahlungsquelle 1 und die UVA Strahlungsquelle 2 nicht in einem gemeinsamen Gehäuse 3 angeordnet sind bietet sich die Verwendung unabhängiger Lichtleiter 7, 8 an. Bei Verwendung eigener Lichtleiter 7 für jeweils die UVB Strahlungsquelle 1 und die UVA Strahlungsquelle 2 wird deren Strahlung, beispielsweise im Handstück 9 oder vor dem Handstück 9, so zusammengeführt, dass diese unmittelbar nebeneinander an der Austrittsfläche 10 bzw. weiteren Austrittsfläche 11 austreten oder an der gleichen Stelle der Austrittsfläche 10 austreten, entsprechend also der Ausgestaltung mit nur einem Lichtleiter 7.

Das aus der Austrittsfläche 10 austretende Licht wird auf zu behandelnde Gebiete 5 der Haut 4 des Patienten geleitet.

Das Verfahren wird vorzugsweise durchgeführt mit der gleichzeitigen Einnahme eines Stoffes durch den Patienten, der die Haut für UV-Therapie empfindlicher macht. Beispielsweise durch Einnahme des Medikamentes Psoralens. Die bevorzugt aufzubringenden Dosis, dies ist die Energie pro Flächeneinheit, wird vorzugsweise so gewählt, dass die UVA-Dosis etwa halb so hoch ist wie die UVB-Dosis.

Bei der Verwendung einer Vorrichtung gemäß Figur 1 zur Durchführung des Verfahrens erfolgt eine Kombinationstherapie zur gleichzeitigen bzw. zeitlich nah aufeinanderfolgenden Bestrahlung im Bereich von 300 bis 320nm sowie im Bereich von UVA-Strahlung bei mehrfacher Minimaler Erythem Dosis (MED). MED bezeichnet eine Energiedosis (kumulierte Energie pro Fläche). MED ist die niedrigste Dosis, bei der sich die Haut bei einer bestimmten Bestrahlung nach einer gewissen Zeit, typischerweise einem Tag, merklich rötet. Sie ist vom Hauttyp, der Jahreszeit und der Wellenlänge der Strahlung abhängig. Die Bestrahlung erfolgt mit einer höheren als der experimentell auf gesunder Haut ermittelten MED. Die Dosis wird daher meist in Vielfachen der MED angegeben. Typisch für die Behandlung der Psoriasis ist bei 308nm etwa eine drei bis sechsfache MED, es wurden aber auch 16-fache MED beschrieben. Die Bestrahlung kann breitbandig aus einer Lampe oder schmalbandig aus einer Lampe oder einem Laser, vorzugsweise einem Laser mit einer Wellenlänge von 351 nm oder 355 nm zur Behandlung von die Haut versorgenden Gefäßen erfolgen. Es kann auch nur UVA-Strahlung lokalisiert angewandt werden, um die gesunde Haut zu schonen.

Die zuvor dargestellte Vorrichtung kann alternativ auch mit nur der UVA Strahlungsquelle 2 allein betrieben werden. Das Verfahren zur Behandlung der Psoriasis beim Menschen wird in diesem Fall so durchgeführt, dass ein gepulster Laser im UVA-Bereich mit einer Wellenlänge zwischen etwa 350 nm und etwa 380 nm, vorzugsweise einer Wellenlänge von 351 oder 355 nm oder einer Wellenlänge im Bereich von etwa 360 nm bis etwa 380 nm verwendet wird. Die Verwendung kann allein oder gemeinsam mit einer UVB-Quelle erfolgen. Damit ist eine lokalisierte Behandlung von Hautkrankheiten, insbesondere der Psoriasis, möglich. Vorzugsweise wird bei der Behandlung die Haut sensibilisiert durch Einnahme eines Medikaments oder Aufbringen einer Creme oder durch ein Bad in einer Lösung einer Substanz wie z. B. Psoralens.

### BEZUGSZEICHENLISTE

- 1: UVB Strahlungsquelle
- 2: UVA Strahlungsquelle
- 3: Gehäuse
- 4: Haut
- 5: Gebiet
- 6: Einkopplung
- 7: Lichtleiter
- 8: Weiterer Lichtleiter
- 9: Handstück
- 10: Austrittsfläche
- 11: Weitere Austrittsfläche

## Patentansprüche

1. Vorrichtung zur Behandlung einer Oberfläche, insbesondere der tierischen oder menschlichen Haut, umfassend eine UVA-Strahlungsquelle (2), deren Strahlung in eine Lichtleiter (7) eingekoppelt wird und eine UVB-Strahlungsquelle (1), deren Strahlung in denselben Lichtleiter (7) oder einen weiteren Lichtleiter (8) eingekoppelt wird, wobei die Strahlung der beiden Strahlungsquellen an einer Austrittsfläche aus dem einen Lichtleiter (7) oder aus den Austrittsflächen der beiden Lichtleiter (7, 8) ausgekoppelt wird,
**dadurch gekennzeichnet, dass**
die UVA-Strahlungsquelle (2) und die UVB-Strahlungsquelle (1) unterschiedliche Strahlungsquellen sind, deren Intensität unabhängig voneinander gewählt werden kann.

2. Vorrichtung nach Anspruch 1, **dadurch gekennzeichnet, dass**
die UVB-Strahlungsquelle (1) Licht einer Wellenlänge von etwa 290 bis 320 nm abgeben kann.

3. Vorrichtung nach Anspruch 1 oder 2, **dadurch gekennzeichnet, dass**
die UVB-Strahlungsquelle (1) und/oder die UVA-Strahlungsquelle (2) ein Laser ist.

4. Vorrichtung nach Anspruch 3, **dadurch gekennzeichnet, dass**
der UVA- und/oder UVB-Laser (1,2) gepulst ist.

5. Vorrichtung nach Anspruch 4, **dadurch gekennzeichnet, dass**
die UVB-Strahlungsquelle (1) ein Laser mit einer Pulslänge etwa 10 bis 300ns ist.

6. Vorrichtung nach einem der Ansprüche 4 oder 5, **dadurch gekennzeichnet, dass**
die UVA-Strahlungsquelle (2) ein Laser mit einer Pulslänge von etwa 10ns bis 300ns oder 10ms bis 100ms ist.

7. Vorrichtung nach einem der Ansprüche 4 bis 6, **dadurch gekennzeichnet, dass**
die Pulse der UVB-Strahlungsquelle (1) und der UVA-Strahlungsquelle (2) als Impulsfolgen abgegeben werden können wobei eine Impulsfolge aus einem oder mehreren Impulsen bestehen kann und die Anzahl der Pulse für jede Strahlungsquelle (1, 2) unterschiedlich sein kann.

8. Vorrichtung nach einem der Ansprüche 3 bis 7, **dadurch gekennzeichnet, dass**
die UVB-Strahlungsquelle (1) ein Laser mit einer Wellenlänge von etwa 308nm ist.

9. Vorrichtung nach einem der Ansprüche 1 bis 8, **dadurch gekennzeichnet, dass**
die UVA-Strahlungsquelle (2) ein UVA-Laser mit einer Wellenlänge größer als etwa 320nm ist.

10. Vorrichtung nach einem der Ansprüche 1 bis 9, **dadurch gekennzeichnet, dass**
die UVA-Strahlungsquelle (2) ein XeF oder Ar-Ionen-Laser mit einer Wellenlänge von etwa 351nm ist.

11. Vorrichtung nach einem der Ansprüche 1 bis 9, **dadurch gekennzeichnet, dass**
die UVA-Strahlungsquelle (2) ein NdYAG Laser mit einer Wellenlänge von etwa 355nm ist.

12. Vorrichtung nach einem der Ansprüche 1 bis 9, **dadurch gekennzeichnet, dass**
die UVA-Strahlungsquelle (2) ein durchstimmbarer Laser mit einer Wellenlänge von etwa 360-430nm ist.

## Claims

1. A device for treating a surface, in particular animal or human skin, comprising a UVA radiation source (2) whose radiation is coupled into an optical waveguide (7), and a UVB radiation source (1) whose radiation is coupled into the same optical waveguide (7) or into a further optical waveguide (8); the radiation of the two radiation sources being coupled out of the one optical waveguide (7) at an exit surface or at the exit surfaces of the two optical waveguides (7, 8),
**characterized in that** the UVA radiation source (2) and the UVB radiation source (1) are different radiation sources whose intensities are selectable independently of each other.

2. The device as recited in Claim 1,
**characterized in that** the UVB radiation source (1) is capable of emitting light having a wavelength of about 290 to 320 nm.

3. The device as recited in Claim 1 or 2,
**characterized in that** the UVB radiation source (1) and/or the UVA radiation source (2) is/are a laser or lasers.

4. The device as recited in Claim 3,
**characterized in that** the UVA and/or UVB laser(s) (1, 2) is/are pulsed.

5. The device as recited in Claim 4,
**characterized in that** the UVB radiation source (1) is a laser with a pulse length of about 10 to 300ns.

6. The device as recited in one of the Claims 4 or 5,
**characterized in that** the UVA radiation source (2) is a laser with a pulse length of about 10ns to 300ns or 10ms to 100ms.

7. The device as recited in one of the Claims 4 trough 6,
**characterized in that** the pulses of the UVB radiation source (1) and UVA radiation source (2) can be emitted as pulse trains; it being possible for a pulse train to be composed of one or more pulses, and for the number of pulses to be different for each radiation source (1, 2).

8. The device as recited in one of the Claims 3 through 7,
**characterized in that** the UVB radiation source (1) is a laser with a wavelength of about 308nm.

9. The device as recited in one of the Claims 1 trough 8,
**characterized in that** the UVA radiation source (2) is a UVA laser with a wavelength greater than 320nm.

10. The device as recited in one of the Claims 1 through 9,
**characterized in that** the UVA radiation source (2) is an XeF or Ar-ion laser with a wavelength of about 351nm.

11. The device as recited in one of the Claims 1 through 9,
**characterized in that** the UVA radiation source (2) is an NdYAG laser with a wavelength of about 355nm.

12. The device as recited in one of the Claims 1 through 9,
**characterized in that** the UVA radiation source (2) is a tunable laser with a wavelength of about 360-430nm.

## Revendications

1. Dispositif pour le traitement d'une surface, notamment de la peau d'un animal ou d'un être humain, comprenant une source de rayonnement UVA (2) dont le rayonnement est injecté dans une fibre optique (7) et une source de rayonnement UVB (1) dont le rayonnement est injecté dans la même fibre optique (7) ou dans une autre fibre optique (8) ; le rayonnement des deux sources de rayonnement étant éjecté sur une surface de sortie d'une fibre optique (7) ou des surfaces de sortie des deux fibres optiques (7, 8),
**caractérisé en ce que** la source de rayonnement UVA (2) et la source de rayonnement UVB (1) sont des sources de rayonnement différentes dont l'intensité peut être choisie de façon indépendante l'une par rapport à l'autre.

2. Dispositif selon la revendication 1, **caractérisé en ce que** la source de rayonnement UVB (1) peut émettre une lumière d'une longueur d'onde d'environ 290 à 320 nm.

3. Dispositif selon la revendication 1 ou 2, **caractérisé en ce que** la source de rayonnement UVB (1) et / ou la source de rayonnement UVA (2) est un laser.

4. Dispositif selon la revendication 3, **caractérisé en ce que** le laser UVA et / ou UVB (1,2) est un laser à impulsions.

5. Dispositif selon la revendication 4, **caractérisé en ce que** la source de rayonnement UVB (1) est un laser avec une longueur d'impulsions d'environ 10 à 300 ns.

6. Dispositif selon une des revendications 4 ou 5, **caractérisé** la source de rayonnement UVA (2) est un laser avec une longueur d'impulsions d'environ 10ns à 300 ns ou 10ms à 100 ms.

7. Dispositif selon une des revendications 4 à 6, **caractérisé en ce que** la pulsation de la source de rayonnement UVB (1) et la source de rayonnement UVA peuvent être émises en tant que trains d'impulsions ; un train d'impulsion pouvant consister en une ou plusieurs impulsions et le nombre d'impulsions pouvant être différent pour chaque source de rayonnement (1, 2).

8. Dispositif selon une des revendications 3 à 7, **caractérisé en ce que** la source de rayonnement UVB (1) est un laser avec une longueur d'onde d'environ 308 nm.

9. Dispositif selon une des revendications 1 à 8, **caractérisé en ce que** la source de rayonnement UVA (2) est un laser UVA avec une longueur d'onde supérieure à environ 320nm.

10. Dispositif selon une des revendications 1 à 9, **caractérisé en ce que** la source de rayonnement UVA (2) est un laser à ions XeF ou Ar avec une longueur d'onde d'environ 351 nm.

11. Dispositif selon une des revendications 1 à 9, **caractérisé en ce que** la source de rayonnement UVA (2) est un laser NdYAG avec une longueur d'onde d'environ 355nm.

12. Dispositif selon une des revendications 1 à 9, **caractérisé en ce que** la source de rayonnement UVA(2) est un laser réglable avec une longueur d'onde d'environ 360-430nm.
